(19) Europäisches Patentamt
European Patent Office
Office européen des brevets

(11) **EP 3 125 838 B1**

(12) **FASCICULE DE BREVET EUROPEEN**

(45) Date de publication et mention
de la délivrance du brevet:
**18.09.2024 Bulletin 2024/38**

(21) Numéro de dépôt: **15717570.4**

(22) Date de dépôt: **27.03.2015**

(51) Classification Internationale des Brevets (IPC):
*A61F 9/00* ^(2006.01)    *B65D 47/18* ^(2006.01)
*B65D 1/02* ^(2006.01)    *B65D 41/04* ^(2006.01)

(52) Classification Coopérative des Brevets (CPC):
**B65D 47/18; B65D 1/023; B65D 1/0246;
B65D 41/0414; B65D 47/2081;** A61F 9/0008

(86) Numéro de dépôt international:
**PCT/FR2015/050796**

(87) Numéro de publication internationale:
**WO 2015/150673 (08.10.2015 Gazette 2015/40)**

(54) **DISPOSITIF DE DISTRIBUTION DE LIQUIDE.**

FLÜSSIGKEITSABGABEVORRICHTUNG

LIQUID DISPENSING DEVICE

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **02.04.2014 FR 1452902**

(43) Date de publication de la demande:
**08.02.2017 Bulletin 2017/06**

(73) Titulaire: **Nemera La Verpilliere
F-38290 La Verpilliere (FR)**

(72) Inventeurs:
• **DECOCK, Thierry
F-69002 Lyon (FR)**
• **QUAGLIA, Benjamin
F-69007 Lyon (FR)**
• **PAINCHAUD, Gaëtan
F-69340 Francheville (FR)**

(74) Mandataire: **LLR
2, rue Jean Lantier
75001 Paris (FR)**

(56) Documents cités:
**BE-A- 648 227        BE-A- 750 714
CH-A5- 597 049        FR-A1- 2 980 378
FR-A5- 2 100 457      US-A- 2 526 225
US-A- 2 742 195       US-A1- 2011 297 703**

**Description**

La présente invention concerne la distribution de liquide, plus particulièrement la distribution dans le domaine pharmaceutique, par exemple du liquide ophtalmique, nasal, buccal ou auriculaire. On entend par « produit liquide » un produit non solide et non gazeux, plus ou moins visqueux.

On connaît déjà, notamment dans la demande de brevet FR2980378 des dispositifs de distribution comprenant un réservoir de stockage du liquide et un embout de distribution, rapporté sur un col du réservoir, par exemple par vissage. Le réservoir et l'embout de distribution sont réalisés en matière plastique. Pour assurer l'étanchéité au liquide du dispositif, une partie de l'embout de distribution est destinée à venir en contact étanche avec la surface interne du col du réservoir de façon à créer une zone d'étanchéité. Ce contact peut être réalisé par un bourrelet annulaire sur le réservoir, ou bien par contact plan sur plan. Le document US 2011/297703 montre un récipient de distribution de colorant alimentaire comprenant une buse.

Il se trouve que le vissage de l'embout de distribution sur le col du réservoir se fait à une haute vitesse (par exemple supérieure à 300 tours par minute), ce qui peut générer des copeaux de matière, issus du frottement de l'embout de distribution avec le col du réservoir au niveau de la zone d'étanchéité. Ces copeaux risquent de tomber dans le liquide contenu dans le réservoir et de le contaminer, ou encore de fausser le fonctionnement de l'embout de distribution. Ce phénomène n'est pas désirable, surtout quand le liquide est du liquide pharmaceutique.

La présente invention vise à proposer un dispositif de distribution garantissant une meilleure conservation et une meilleure distribution du liquide.

A cet effet, l'invention a pour objet un dispositif de distribution de liquide, conforme à la revendication 1 comprenant un embout de distribution et un col d'un réservoir de stockage du liquide, l'embout de distribution étant rapporté sur le col,

- le col présentant une surface interne de forme générale sensiblement tubulaire,
- l'embout de distribution présentant une jupe interne de forme générale sensiblement tubulaire montée à l'intérieur du col du réservoir et définissant avec la surface interne du col au moins une zone d'étanchéité annulaire, de façon que du liquide se trouvant dans le réservoir ne puisse pas passer entre la jupe interne de l'embout de distribution et le col du réservoir,
- la jupe interne de l'embout de distribution définissant, avec la surface interne du col, une zone de retenue de copeaux, distincte et disposée en amont de la zone d'étanchéité par rapport au réservoir, cette zone de retenue étant configurée de sorte que des copeaux formés au niveau de la zone d'étanchéité au cours de l'assemblage de l'embout de distribution sur le col du réservoir ne puissent pas se retrouver dans le réservoir.

Grâce à la zone de retenue de copeaux, il est possible de bloquer toutes les particules qui peuvent être créées lors de l'assemblage, résultant de frottements entre l'intérieur du réservoir et l'embout, donc d'éviter que ces particules ne tombent dans le réservoir et contaminent le liquide contenu dans le réservoir, ou encore altèrent le fonctionnement de l'embout de distribution, par exemple en bouchant des canaux. Ainsi, la zone de retenue de copeaux forme une barrière disposée entre la zone d'étanchéité et le réservoir pour retenir les éventuels copeaux qui seraient formés lorsque la surface interne du col du réservoir est mise en accostage avec la jupe interne de l'embout de distribution.

On notera que l'assemblage de l'embout de distribution sur le col du réservoir peut se faire de différentes façons, notamment par vissage, emmanchement à force, ou encore par encliquetage. La zone de retenue est particulièrement avantageuse dans le cas où l'assemblage de l'embout de distribution sur le réservoir est effectué de façon très rapide, tout particulièrement lors d'un vissage à haute vitesse, par exemple supérieure à 300 tours par minute. En effet, lors d'un assemblage rapide, davantage de copeaux peuvent se former au niveau de la zone d'étanchéité, car la matière n'a pas le temps de se déformer pour compenser le serrage générant la zone d'étanchéité. On comprend que dans le cas avantageux où l'embout de distribution est rapporté sur le col par vissage, pour assurer ce vissage, l'embout de distribution comprend une surface filetée et le col du réservoir comprend une surface taraudée complémentaire.

On notera que le dispositif de distribution est particulièrement intéressant car on aurait pu songer, de façon alternative, à effectuer des changements de matériaux pour réaliser le réservoir et l'embout de distribution. Néanmoins, les résultats obtenus avec le dispositif présenté ci-dessus sont plus intéressants pour éviter des copeaux. En effet, par exemple en utilisant un flacon en polypropylène, on obtient un flacon plus rigide dont l'utilisation est plus difficile et dont le coût est plus élevé.

On notera par ailleurs que la zone de retenue de copeaux est distincte de la zone d'étanchéité. En effet, cette zone de retenue a pour fonction de s'interposer entre le réservoir et la zone d'étanchéité où des copeaux risquent d'être créés, et n'a donc pas pour fonction première d'assurer une étanchéité au liquide, même s'il n'est pas exclu qu'elle puisse le faire. On entend par « zone de retenue de copeaux distincte de la zone d'étanchéité » le fait que la juxtaposition des deux zones se distingue d'une surface exactement cylindrique et plane. Par ailleurs, on entend par « copeaux » des particules de toute forme, susceptibles d'être créées par l'embout de distribution et/ou le réservoir lors de l'assemblage des deux pièces, notamment par vissage. Les copeaux sont par exemple sous forme de filaments ou de poussières.

- Selon l'invention, la surface interne du col du réservoir a un diamètre général, elle comprend une forme annulaire convexe faisant saillie de la surface interne du col, de façon à définir un diamètre diminué par rapport au diamètre général du col du réservoir et ainsi former, par coopération avec la jupe interne de l'embout de distribution, la zone de retenue des copeaux. Ainsi, une proéminence formant une inflexion est réalisée sur la surface interne du col de façon à retenir des copeaux issus du vissage de l'embout de distribution sur le réservoir. De préférence, la forme annulaire convexe présente une inflexion douce par rapport à la surface interne du col afin de ne pas créer de nouveaux copeaux au niveau de la zone de retenue des copeaux lors du contact qui se réalise. Par ailleurs, le diamètre diminué est de préférence inférieur au diamètre du col du réservoir au niveau de la zone d'étanchéité. On notera qu'il est particulièrement intéressant de réaliser ces variations de diamètre sur la surface interne du col du réservoir plutôt que sur la jupe interne de l'embout de distribution, car cela permet de faciliter l'assemblage des deux pièces. L'invention peut en outre comporter l'une ou plusieurs des caractéristiques suivantes, prises seules ou en combinaison.

- Le dispositif est configuré de telle sorte que, lors de l'assemblage de l'embout de distribution sur le col du réservoir, la jupe interne de l'embout de distribution entre en contact avec la surface interne du col du réservoir d'abord au niveau de la zone d'étanchéité, puis au niveau de la zone de retenue des copeaux. Ainsi, le contact de la zone de retenue s'effectue à la fin de l'assemblage, moment au cours duquel la force d'assemblage est plus petite, en particulier le couple de vissage lors d'un assemblage par vissage. Ainsi, on ne crée pas de copeau entre la surface interne du col du réservoir et la jupe de l'embout de distribution au niveau de la zone de retenue des copeaux. En outre, la formation de la zone d'étanchéité se fait en priorité, si bien que l'on garantit que cette zone d'étanchéité sera formée, quelles que soient les tolérances de fabrication des pièces.

- Le col du réservoir présente une longueur $L_{col\ min}$ délimitée par l'extrémité supérieure de la longueur du col assurant l'étanchéité $L_{étanchéité\ col}$ et l'extrémité supérieure de la longueur du col assurant la zone de retenue des copeaux $L_{retenue\ col}$, l'embout de distribution présente une longueur $L_{embout\ max}$ délimitée par l'extrémité inférieure de la longueur de l'embout assurant l'étanchéité $L_{étanchéité\ embout}$ et l'extrémité inférieure de la longueur de l'embout assurant la zone de retenue des copeaux $L_{retenue\ embout}$, le dispositif est tel que $L_{col\ min} > L_{embout\ max}$. Grâce à cette configuration, lors de l'assemblage de l'embout de distribution sur le col du réservoir, la jupe interne de l'embout de distribution entre en contact avec la surface interne du col du réservoir d'abord au niveau de la zone d'étanchéité, puis au niveau de la zone de retenue des copeaux.

- La surface interne du col du réservoir au niveau de la zone d'étanchéité est sensiblement plane, l'étanchéité avec la jupe interne de l'embout de distribution se faisant par un contact plan sur plan de la jupe interne de l'embout de distribution avec la surface interne du col du réservoir. On entend ici par « contact plan sur plan » tout type de contact d'étanchéité réalisé entre deux surfaces planes de révolution, tel qu'un contact cylindre contre cylindre, cylindre contre cône, cône contre cône. On notera que dans le cas d'un tel contact plan sur plan pour réaliser l'étanchéité, la force d'assemblage peut être relativement élevée car la surface de frottement est plus grande. Toutefois, comme le contact se réalise de façon progressive, le risque de création des copeaux est diminué.

- La surface interne du col du réservoir au niveau de la zone d'étanchéité comprend un bourrelet annulaire.

- La surface interne du col du réservoir comprend, en aval et au voisinage direct de la zone d'étanchéité, un évidement annulaire. Cet évidement permet que la surface de contact dans la zone d'étanchéité soit la plus ponctuelle possible, de façon à créer moins de copeaux. En outre, cet évidement permet de réaliser une partie supérieure du col présentant une épaisseur minimale, susceptible de former une butée avec le fond d'une gorge formée par la jupe interne de l'embout de distribution. Comme cette épaisseur est minimale, la butée entre le haut du col du réservoir et le fond de cette gorge est plus franche, ce qui permet de créer une seconde zone d'étanchéité au liquide particulièrement efficace.

- Le col du réservoir est réalisé en polyéthylène basse densité (LDPE, abréviation en anglais du terme « low density polyethylene ») et la jupe interne de l'embout de distribution est réalisée en polyéthylène haute densité (HDPE, abréviation en anglais du terme « high density polyethylene »). Ainsi, le réservoir est réalisé dans un matériau souple, permettant de distribuer aisément le liquide par pression de l'utilisateur, et l'embout de distribution est réalisé dans un matériau plus rigide, ce qui permet de faciliter l'assemblage des pièces et d'assurer une étanchéité efficace du dispositif de distribution. Dans ce cas, les copeaux sont principalement créés sur le réservoir et la zone de retenue proposée dans le présent dispositif est particulièrement intéressante.

- L'extrémité de la jupe interne de l'embout de distribution située du côté du réservoir présente une forme en entonnoir. Ceci a pour effet de faciliter l'assemblage de l'embout de distribution avec le réservoir.

L'invention sera mieux comprise à la lecture de la description qui va suivre, donnée uniquement à titre d'exemple et faite en se référant aux dessins dans lesquels :

- la figure 1 est une vue en coupe longitudinale d'un dispositif selon un premier mode de réalisation,
- la figure 2 est une vue similaire à la figure 1, d'un dispositif selon un deuxième mode de réalisation,
- la figure 3 est une vue similaire à la figure 1, d'un dispositif selon un troisième mode de réalisation,
- la figure 4 est une vue schématique illustrant les positions relatives des zones d'étanchéité et des zones de retenue des copeaux, et
- la figure 5 est une vue en coupe longitudinale du réservoir du dispositif de la figure 3.

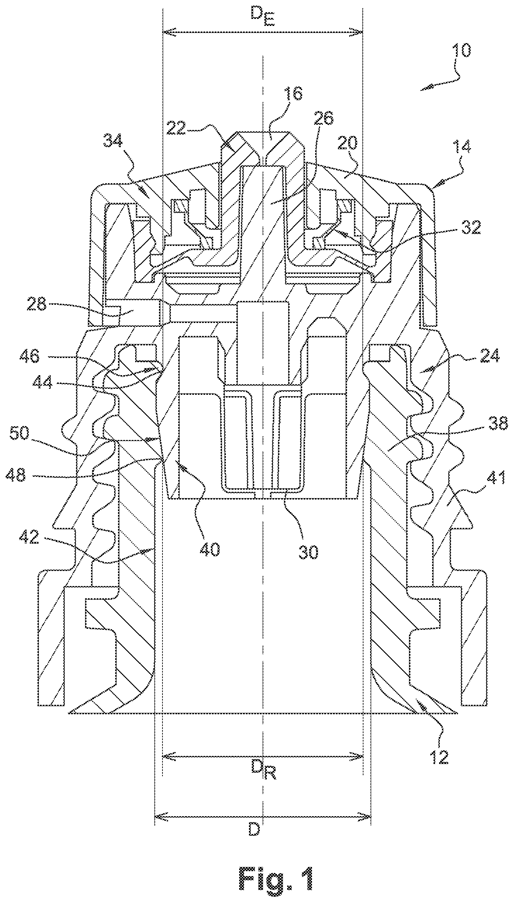

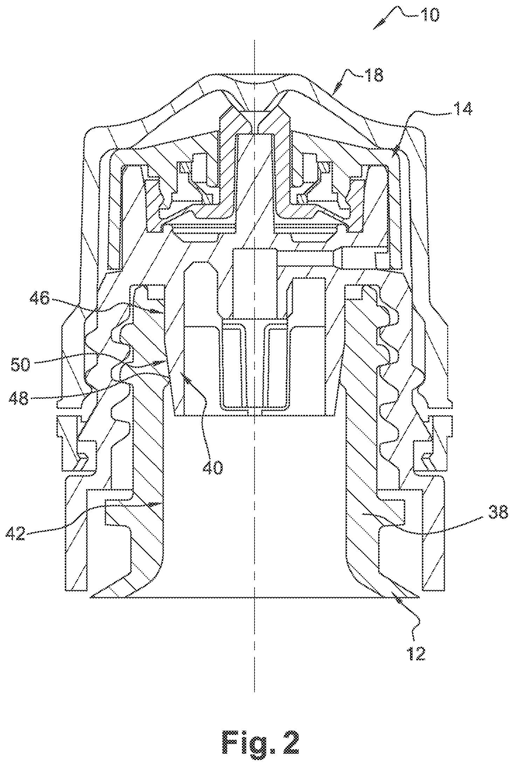

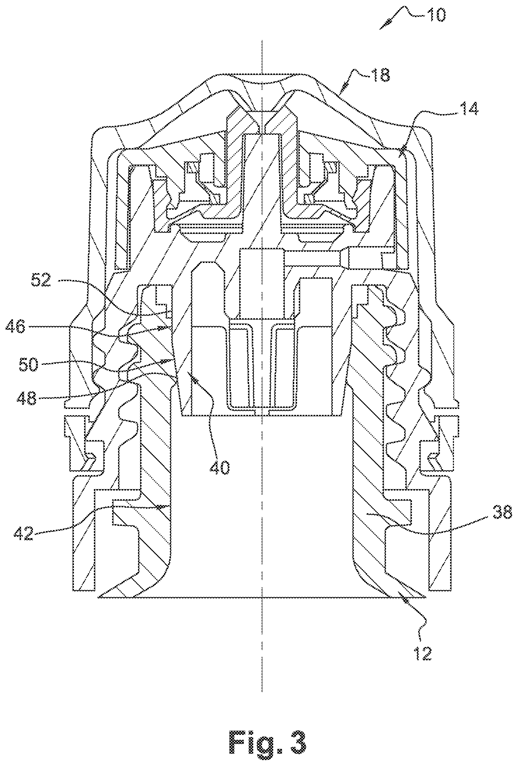

On a représenté sur la figure 1 un dispositif 10 de distribution de liquide sous forme de gouttes comprenant un réservoir 12, sur lequel est rapporté un embout de distribution 14, qui peut être surmonté d'un capuchon de protection 18, visible sur la figure 2 ou la figure 3. Le réservoir 12 comprend un col 38 pour recevoir l'embout de distribution 14.

L'embout de distribution 14 présente une extrémité supérieure de distribution 20 sur laquelle est ménagé un orifice 16 de distribution des gouttes calibrées, dans cet exemple, par des moyens 16 de formation de gouttes. Plus précisément, toujours dans cet exemple, l'embout de distribution 14 est de forme générale sensiblement cylindrique. L'embout de distribution 14 comprend ici une valve 22 de distribution de gouttes, réalisée en matériau élastomère de façon à prendre, sous la pression du liquide (lorsque l'utilisateur appuie sur le réservoir 12), une configuration de passage du liquide. L'embout de distribution 14 comprend par ailleurs un support de valve 24, rapporté sur le réservoir 12, portant un pion 26 formant un siège contre lequel la valve 22 est plaquée et comportant également un conduit 28 de passage d'air, obturé par un organe 30 perméable à l'air empêchant l'introduction de bactéries dans le réservoir 12. L'embout de distribution 14 comporte également un élément de rappel 32, permettant de rappeler la valve 22 en configuration de blocage du liquide. Enfin, l'embout de distribution 14 comprend un capot 34 de plaquage de la valve 22 contre le support 24.

Plus précisément, le support de valve 24 comprend une jupe interne 40 de forme générale sensiblement tubulaire, montée à l'intérieur du col 38 du réservoir 12, ainsi qu'une jupe externe 41 entourant le col 38 du réservoir 12 et présentant par exemple une surface taraudée pour assurer le vissage de l'embout de distribution 14 sur le réservoir 12. La surface taraudée se trouve du côté intérieur de la jupe externe 41. La jupe interne 40 présente une extrémité inférieure de forme en entonnoir, afin de faciliter l'introduction de l'embout de distribution 14 dans le col 38 du réservoir 12. Dans le dispositif 10 de la figure 1, le col 38 présente une surface interne 42 sensiblement tubulaire, ayant un diamètre général et qui comprend un bourrelet annulaire 44. Le bourrelet annulaire 44 forme une zone d'étanchéité 46 avec la jupe interne 40 de l'embout de distribution 14. Lors du vissage de l'embout de distribution 14, la jupe interne 40 vient en contact avec le bourrelet annulaire 44 à une haute vitesse, ce qui peut générer des copeaux de matière plastique, par exemple sous forme de filaments, qui risquent de tomber dans le réservoir 12 et contaminer le liquide contenu dans le réservoir 12.

La surface interne 42 du col 38 comprend par ailleurs une forme annulaire convexe 48, faisant saillie de la surface interne 42 du col 38, de façon à définir un diamètre diminué par rapport au diamètre général, appelé diamètre $D_R$ de retenue de copeaux, représenté sur la figure 1. Ce diamètre $D_R$ de retenue de copeaux est inférieur au diamètre général D du col 38 du réservoir 12, ainsi qu'au diamètre $D_E$ du col 38 du réservoir 12 au niveau de la zone d'étanchéité 46, appelé diamètre d'étanchéité $D_E$. Cette forme convexe 48 réalise, par coopération avec la jupe interne 40 de l'embout de distribution 14, une zone de retenue de copeaux 50. Lorsque l'embout de distribution 14 est monté sur le col 38 du réservoir 12, par vissage, les copeaux créés par frottement entre le col 38 et la jupe 40 dans la zone d'étanchéité 46 sont bloqués dans cette zone de retenue des copeaux 50 et ne peuvent pas tomber dans le réservoir 12. On comprend que le col 38 du réservoir présente une surface externe filetée pour assurer le vissage de l'embout de distribution 14 sur le réservoir 12, par coopération avec la surface taraudée de la jupe externe 41.

Dans le mode de réalisation sur la figure 2, la surface interne 42 du col 38 ne comprend pas un bourrelet annulaire mais est sensiblement plane dans la zone d'étanchéité 46. Dans l'exemple, l'étanchéité de la surface interne 42 avec la jupe interne 40 se fait par un contact plan sur plan, en l'occurrence un contact cylindre contre cylindre des deux surfaces tubulaires de la jupe 40 et de la surface interne 42. La surface interne 42 du col 38 comprend également une forme annulaire convexe 48 faisant saillie de la surface interne 42, de façon à former, par coopération avec la jupe interne 40 de l'embout de distribution 14, une zone de retenue des copeaux 50. Dans ce cas, la surface de contact est plus étendue, sans contact pointu, le risque de création des copeaux est ainsi diminué.

Sur la figure 3, l'étanchéité de la surface interne 42 avec la jupe interne 40 est également assurée par un contact cylindre contre cylindre. A la différence de l'exemple illustré sur la figure 2, la surface interne 42 du col 38 comprend, en aval et au voisinage direct de la zone d'étanchéité 46, un évidement annulaire 52. Comme les copeaux sont créés dans la zone d'étanchéité 46, il est préférable que la surface de contact dans la zone d'étanchéité 46 soit la plus ponctuelle possible. Ainsi, le couple de vissage sera réduit et l'on créera moins de copeaux.

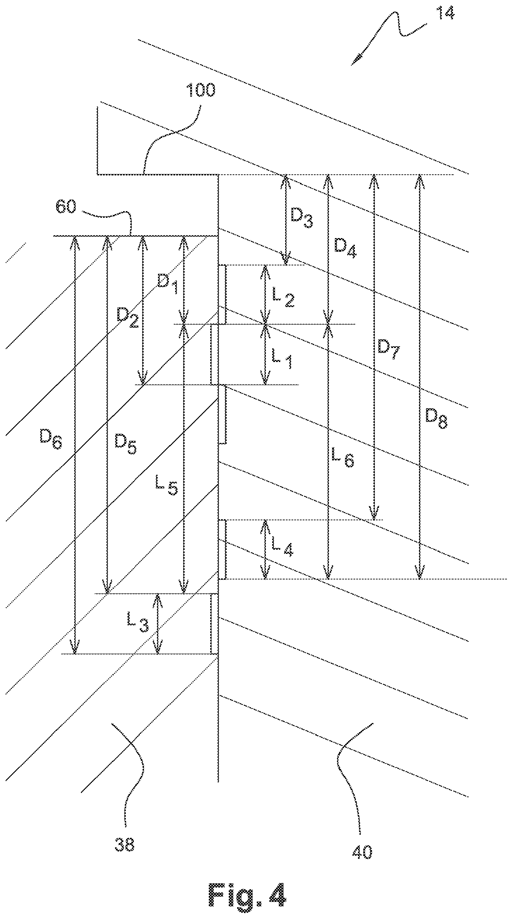

Lors de l'assemblage de l'embout de distribution 14 sur le réservoir 12, la jupe interne 40 est introduite dans le col 38 du réservoir 12, le contact de la zone de retenue 50 s'effectue après le contact de la zone d'étanchéité 46. Afin d'illustrer la configuration des pièces pour assurer cet ordre de contact, une vue schématique illustrant les positions relatives des zones d'étanchéité 46 et des zones de retenue des copeaux 50 est représenté sur la figure 4.

La surface interne 42 du col 38 et la jupe interne 40 présentent chacune une longueur théorique dans la direction longitudinale L1 = $L_{\text{étanchéité théorique col}}$ et L2 = $L_{\text{étanchéité théorique embout}}$ prévues pour assurer l'étanchéité, en réalisant la zone d'étanchéité 46.

La longueur théorique du col 38 L1 = $L_{\text{étanchéité théorique col}}$ définit, en référence à l'extrémité supérieure 60 du col 38, une distance théorique minimale D1 = $D_{\text{étanchéité théorique col min}}$ et une distance théorique maximale D2 = $D_{\text{étanchéité théorique col max}}$.

La longueur théorique de l'embout de distribution 14 L2 = $L_{\text{étanchéité théorique embout}}$ définit, en référence au fond de la gorge 100 de l'embout de distribution 14, une distance théorique minimale D3 = $D_{\text{étanchéité théorique embout min}}$ et une distance théorique maximale D4 = $D_{\text{étanchéité théorique embout max}}$.

Pour qu'il y ait l'étanchéité, c'est-à-dire qu'au moins une partie de la longueur L1 = $L_{\text{étanchéité théorique col}}$ de la surface interne 42 du col 38 soit en contact avec au moins une partie de longueur L2 = $L_{\text{étanchéité théorique embout}}$ de la jupe interne 40 de l'embout de distribution 14, il faut que l'on ait à la fois :

$$\text{i)} \quad D1 = D_{\text{étanchéité théorique col min}} < D_{\text{étanchéité théorique embout max}} = D4 \text{ et que}$$

et que

$$\text{ii)} \quad D3 = D_{\text{étanchéité théorique embout min}} < D_{\text{étanchéité théorique col max}} = D2.$$

Ainsi, lorsque l'extrémité du col 38 et le fond de la gorge 100 de l'embout de distribution 14 sont en contact, l'étanchéité est garantie.

De même, on peut comprendre les conditions pour qu'il y ait existence de la zone de retenue de copeaux 50. La surface interne 42 du col 38 et la jupe interne 40 présentent chacune une longueur théorique dans la direction longitudinale L3 = $L_{\text{retenue théorique col}}$ et L4 = $L_{\text{retenue théorique embout}}$ prévues pour assurer la zone de retenue, en réalisant la zone de retenue de copeaux 50.

La longueur théorique du col 38 L3 = $L_{\text{retenue théorique col}}$ définit, en référence à l'extrémité supérieure 60 du col 38, une distance théorique minimale D5 = $D_{\text{retenue théorique col min}}$ et une distance théorique maximale D6 = $D_{\text{retenue théorique col max}}$.

La longueur théorique de l'embout de distribution 14 L4 = $L_{\text{retenue théorique embout}}$ définit, en référence au fond de la gorge 100 de l'embout de distribution 14, une distance théorique minimale D7 = $D_{\text{retenue théorique embout min}}$ et une distance théorique maximale D8 = $D_{\text{retenue théorique embout max}}$.

Pour qu'il y ait la zone de retenue, c'est-à-dire pour qu'au moins une partie de la longueur L3 = $L_{\text{retenue théorique col}}$ de la surface interne 42 du col 38 soit en contact avec au moins une partie de longueur L4 = $L_{\text{retenue théorique embout}}$ de la jupe interne 40 de l'embout de distribution 14, il faut que l'on ait à la fois :

$$\text{iii)} \quad D5 = D_{\text{étanchéité théorique col min}} < D_{\text{étanchéité théorique embout max}} = D8 \text{ et que}$$

et que

$$\text{iv)} \quad D7 = D_{\text{étanchéité théorique embout min}} < D_{\text{étanchéité théorique col max}} = D6.$$

Ainsi, lorsque l'extrémité du col 38 et le fond de gorge 100 de l'embout de distribution de distribution 14 sont en contact, la zone de retenue de copeaux 50 est garantie.

Dans la suite, on développe les configurations permettant que le contact de la zone de retenue 50 s'effectue après le contact de la zone d'étanchéité 46.

Les distances théoriques minimales sur le col D1 = $D_{\text{étanchéité théorique col min}}$ et D5 = $D_{\text{retenue théorique col min}}$ définissent une longueur du col L5 = $L_{\text{col min}}$, délimitée par l'extrémité supérieure de la longueur théorique assurant l'étanchéité et celle de la longueur théorique assurant la zone de retenue. Par ailleurs, les distances théoriques maximales sur l'embout D4 = $D_{\text{étanchéité théorique embout max}}$ et D8 = $D_{\text{retenue théorique embout max}}$ définissent une longueur de l'embout L6 = $L_{\text{embout max}}$ délimitée par l'extrémité inférieure de la longueur théorique assurant l'étanchéité et celle de la longueur théorique assurant la zone de retenue.

Pour que le contact de la zone de retenue 50 s'effectue après le contact de la zone d'étanchéité 46, il faut que l'on ait : L5 = $L_{\text{col min}}$ > L6 = $L_{\text{embout max}}$.

On comprend qu'une longueur « théorique » désigne une longueur prédéfinie sur le col du réservoir ou l'embout de distribution au moment de la conception ou de la fabrication de ces pièces, sachant que, compte-tenu des tolérances de fabrication, une fois les pièces assemblées, l'étanchéité ou la retenue des copeaux ne s'effectue pas forcément sur toute la longueur théorique, mais seulement sur une partie de cette longueur. Ainsi, sur les dispositifs configurés selon ce mode de réalisation, on peut constater que le col du réservoir présente une longueur $L_{\text{col min}}$ délimitée par l'extrémité supérieure de la longueur du col assurant l'étanchéité $L_{\text{étanchéité col}}$ et l'extrémité supérieure de la longueur du col assurant la zone de retenue des copeaux $L_{\text{retenue col}}$, l'embout de distribution présente une longueur $L_{\text{embout max}}$ délimitée par l'extrémité inférieure de la longueur de l'embout de distribution assurant l'étanchéité $L_{\text{étanchéité embout}}$ et l'extrémité inférieure de la longueur de l'embout de distribution assurant la zone de retenue des copeaux $L_{\text{retenue embout}}$, et le dispositif est dimensionné de sorte que $L_{\text{col min}}$ > $L_{\text{embout max}}$.

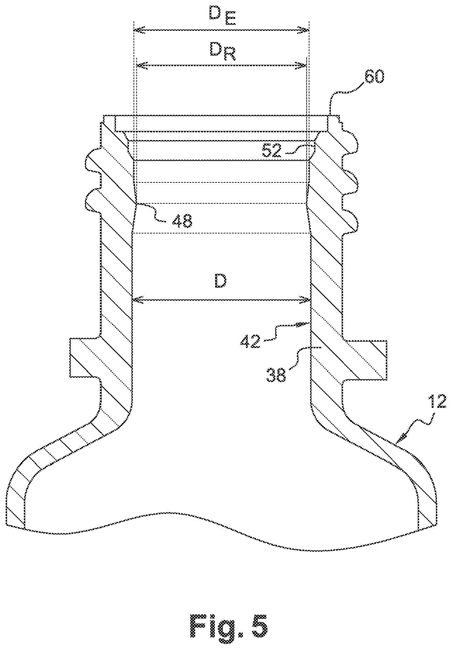

Comme on peut le voir sur la figure 5, le réservoir 12 est configuré de sorte que la surface interne 42 du col 38 a un diamètre d'étanchéité $D_E$ supérieur au diamètre de retenue $D_R$ de copeaux.

On notera que le col 38 du réservoir 12 est réalisé, dans cet exemple, en polyéthylène basse densité, et la jupe interne 42 de l'embout de distribution 14 est réalisée en polyéthylène haute densité.

On comprend que les dispositifs 10 décrits ci-dessus sont particulièrement intéressants pour éviter la présence de copeaux dans le réservoir 12 et que l'invention n'est pas limitée aux exemples décrits ci-dessus.

## Revendications

**1.** Dispositif (10) de distribution de liquide, comprenant un embout de distribution (14) et un col (38) d'un réservoir (12) de stockage du liquide, l'embout de distribution (14) étant rapporté sur le col (38) par vissage,

- le col (38) présentant une surface interne de forme générale sensiblement tubulaire,
- l'embout de distribution (14) présentant une jupe interne (40) de forme générale sensiblement tubulaire, montée à l'intérieur du col (38) du réservoir (12) et définissant avec la surface interne (42) du col (38) au moins une zone d'étanchéité (46) annulaire, de façon que du liquide se trouvant dans le réservoir (12) ne puisse pas passer entre la jupe interne (40) de l'embout de distribution (14) et le col (38) du réservoir (12),
**caractérisé en ce que** la jupe interne (40) de l'embout de distribution (14) définit, avec la surface interne (42) du col (38), une zone de retenue de copeaux (50), distincte et disposée en amont de la zone d'étanchéité (46) par rapport au réservoir (12), cette zone de retenue de copeaux (50) étant configurée de sorte que des copeaux formés au niveau de la zone d'étanchéité (46) au cours de l'assemblage de l'embout de distribution (14) sur le col (38) du réservoir (12) ne puissent pas se retrouver dans le réservoir (12),
et **en ce que** la surface interne (42) du col (38) du réservoir (12), ayant un diamètre général, comprend une forme annulaire convexe (48) faisant saillie de la surface interne (42) du col (38), de façon à définir un diamètre diminué ($D_R$) par rapport au diamètre général (D) du col (38) du réservoir (12) et ainsi former, par coopération avec la jupe interne (40) de l'embout de distribution (14), la zone de retenue des copeaux (50).

**2.** Dispositif (10) selon la revendication précédente, dans lequel le diamètre diminué ($D_R$) est inférieur au diamètre ($D_E$) du col (38) du réservoir (12) au niveau de la zone d'étanchéité (46).

**3.** Dispositif (10) selon l'une quelconque des revendications précédentes, configuré de telle sorte que lors de l'assemblage de l'embout de distribution (14) sur le col (38) du réservoir (12), la jupe interne (40) de l'embout de distribution (14) entre en contact avec la surface interne (42) du col (38) du réservoir (12) d'abord au niveau de la zone d'étanchéité (46), puis au niveau de la zone de retenue des copeaux (50).

**4.** Dispositif (10) selon la revendication précédente, dans lequel

- le col (38) du réservoir (12) présente une longueur $L_{\text{col min}}$ (L5) délimitée par l'extrémité supérieure de la longueur du col assurant l'étanchéité $L_{\text{étanchéité col}}$ (L1) et l'extrémité supérieure de la longueur du col assurant la zone de retenue des copeaux (50) $L_{\text{retenue col}}$ (L3),
- l'embout de distribution (14) présente une longueur $L_{\text{embout max}}$ (L6) délimitée par l'extrémité inférieure de la longueur de l'embout assurant l'étanchéité $L_{\text{étanchéité embout}}$ (L2) et l'extrémité inférieure de la longueur de l'embout assurant la zone de retenue des copeaux (50) L$_{retenue\ embout}$ (L4),

et dans lequel L$_{col\ min}$ > L$_{embout\ max.}$ (L5 > L6).

5. Dispositif (10) selon l'une quelconque des revendications précédentes, dans lequel la surface interne (42) du col (38) du réservoir (12) au niveau de la zone d'étanchéité (46) est sensiblement plane, l'étanchéité avec la jupe interne (40) de l'embout de distribution (14) se faisant par un contact plan sur plan de la jupe interne (40) de l'embout de distribution (14) avec la surface interne (42) du col (38) du réservoir (12).

6. Dispositif (10) selon l'une quelconque des revendications 1 à 2, dans lequel la surface interne (42) du col (38) du réservoir (12) au niveau de la zone d'étanchéité (46) comprend un bourrelet annulaire (44).

7. Dispositif (10) selon l'une quelconque des revendications précédentes, dans lequel la surface interne (42) du col (38) du réservoir (12) comprend, en aval et au voisinage direct de la zone d'étanchéité (46), un évidement annulaire (52).

8. Dispositif (10) selon l'une quelconque des revendications précédentes, dans lequel le col (38) du réservoir (12) est réalisé en polyéthylène basse densité (LDPE) et la jupe interne (42) de l'embout de distribution (14) est réalisée en polyéthylène haute densité (HDPE).

9. Dispositif (10) selon l'une quelconque des revendications précédentes, dans lequel l'extrémité de la jupe interne (40) de l'embout de distribution (14) située du côté du réservoir (12) présente une forme en entonnoir.

**Patentansprüche**

1. Vorrichtung (10) zur Abgabe von Flüssigkeit, aufweisend ein Abgabeendstück (14) und einen Hals (38) eines Behälters (12) zur Lagerung der Flüssigkeit, wobei das Abgabeendstück (14) durch Verschraubung auf den Hals (38) aufgesetzt ist,

   - der Hals (38) eine Innenfläche mit einer im Wesentlichen röhrenförmigen allgemeinen Form aufweist,
   - das Abgabeendstück (14) eine Innenschürze (40) von allgemein im Wesentlichen röhrenförmiger Gestalt aufweist, die im Inneren des Halses (38) des Behälters (12) angebracht ist und mit der Innenfläche (42) des Halses (38) mindestens einen ringförmige Dichtungsbereich (46) definiert, so dass Flüssigkeit, die sich im Behälter (12) befindet, nicht zwischen der Innenschürze (40) des Abgabeendstücks (14) und dem Hals (38) des Behälters (12) hindurchtreten kann,
   **dadurch gekennzeichnet, dass** die Innenschürze (40) des Abgabeendstücks (14) zusammen mit der Innenfläche (42) des Halses (38) einen Spanrückhaltebereich (50) definiert, der sich von dem Dichtungsbereich (46) unterscheidet und in Bezug auf den Behälter (12) stromaufwärts angeordnet ist, wobei dieser Spanrückhaltebereich (50) so konfiguriert ist, dass Späne, die während der Montage des Abgabeendstücks (14) auf dem Hals (38) des Behälters (12) an dem Dichtungsbereich (46) gebildet werden, nicht in den Behälter (12) gelangen können,
   und dadurch, dass die Innenfläche (42) des Halses (38) des Behälters (12), die einen allgemeinen Durchmesser hat, eine konvexe Ringform (48) aufweist, die von der Innenfläche (42) des Halses (38) vorsteht, um einen verringerten Durchmesser (DR) in Bezug auf den allgemeinen Durchmesser (D) des Halses (38) des Behälters (12) zu definieren und dadurch durch Zusammenwirken mit der Innenschürze (40) des Abgabeendstücks (14) den Spanrückhaltebereich (50) zu bilden.

2. Vorrichtung (10) nach dem vorhergehenden Anspruch, wobei der verringerte Durchmesser (D$_R$) kleiner ist als der Durchmesser (D$_E$) des Halses (38) des Behälters (12) an dem Dichtungsbereich (46).

3. Vorrichtung (10) nach einem der vorhergehenden Ansprüche, die so konfiguriert ist, dass bei der Montage des Abgabeendstücks (14) auf dem Hals (38) des Behälters (12) die Innenschürze (40) des Abgabeendstücks (14) die Innenfläche (42) des Halses (38) des Behälters (12) zunächst im Bereich des Dichtungsbereichs (46) und dann im Bereich des Spanrückhaltebereichs (50) berührt.

4. Vorrichtung (10) nach dem vorhergehenden Anspruch, wobei

- der Hals (38) des Behälters (12) eine Länge $L_{Hals\ min}$ (L5) aufweist, die durch das obere Ende der Länge des Halses, der die Abdichtung $L_{Abdichtung\ Hals}$ (L1) gewährleistet, und das obere Ende der Länge des Halses, der den Spanrückhaltebereich (50) gewährleistet $L_{Rüekhalte\ Hals}$ (L3), begrenzt wird.
- das Abgabeendstück (14) eine Länge $L_{Endstück\ max}$ (L6) aufweist, die durch das untere Ende der Länge des Endstücks, die die Abdichtung $L_{Abdichtung\ Endstück}$ (L2) gewährleistet, und das untere Ende der Länge des Endstücks, das den Spanrückhaltebereich (50) gewährleistet $L_{Rüekhalte\ Endstück}$ (L4), begrenzt wird,

und wobei $L_{Hals\ min} > L_{Endstück\ max}$, (L5 > L6).

**5.** Vorrichtung (10) nach einem der vorhergehenden Ansprüche, wobei die Innenfläche (42) des Halses (38) des Behälters (12) im Bereich des Dichtungsbereichs (46) im Wesentlichen eben ist, wobei die Abdichtung mit der Innenschürze (40) des Abgabeendstücks (14) durch einen Plan-auf-Plan-Kontakt der Innenschürze (40) des Abgabeendstücks (14) mit der Innenfläche (42) des Halses (38) des Behälters (12) erfolgt.

**6.** Vorrichtung (10) nach einem der Ansprüche 1 bis 2, wobei die Innenfläche (42) des Halses (38) des Behälters (12) im Bereich des Dichtungsbereichs (46) einen ringförmigen Wulst (44) aufweist.

**7.** Vorrichtung (10) nach einem der vorhergehenden Ansprüche, wobei die Innenfläche (42) des Halses (38) des Behälters (12) stromabwärts und in direkter Nachbarschaft zu dem Dichtungsbereich (46) eine ringförmige Aussparung (52) aufweist.

**8.** Vorrichtung (10) nach einem der vorhergehenden Ansprüche, wobei der Hals (38) des Behälters (12) aus Polyethylen niedriger Dichte (LDPE) und die Innenschürze (42) des Abgabeendstücks (14) aus Polyethylen hoher Dichte (HDPE) hergestellt ist.

**9.** Vorrichtung (10) nach einem der vorhergehenden Ansprüche, wobei das Ende der Innenschürze (40) des Abgabeendstücks (14), das sich auf der Seite des Behälters (12) befindet, eine Trichterform aufweist.

## Claims

**1.** Liquid dispensing device (10), comprising a dispensing end piece (14) and a neck (38) of a reservoir (12) for storing the liquid, the dispensing end piece (14) being attached to the neck (38) by screwing,

- the neck (38) having an internal surface of substantially tubular general shape,
- the dispensing end piece (14) having an internal skirt (40) of substantially tubular general shape which is mounted inside the neck (38) of the reservoir (12) and which defines, with the internal surface (42) of the neck (38), at least one annular sealing zone (46) such that liquid present in the reservoir (12) cannot pass between the internal skirt (40) of the dispensing end piece (14) and the neck (38) of the reservoir (12),

**characterized in that** the internal skirt (40) of the dispensing end piece (14) defines, with the internal surface (42) of the neck (38), a separate chips retention zone (50) arranged upstream of the sealing zone (46) with respect to the reservoir (12), this chips retention zone (50) being configured such that chips formed at the sealing zone (46) while the dispensing end piece (14) is being assembled on the neck (38) of the reservoir (12) cannot get into the reservoir (12),

and **in that** the internal surface (42) of the neck (38) of the reservoir (12) has a general diameter and comprises a convex annular shape (48) projecting from the internal surface (42) of the neck (38) so as to define a reduced diameter ($D_R$) with respect to the general diameter (D) of the neck (38) of the reservoir (12) and thus to form, by cooperation with the internal skirt (40) of the dispensing end piece (14), the chips retention zone (50).

**2.** Device (10) as claimed in the preceding claim, wherein the reduced diameter ($D_R$) is smaller than the diameter ($D_E$) of the neck (38) of the reservoir (12) at the sealing zone (46).

**3.** Device (10) as claimed in any one of the preceding claims, configured such that, when assembling the dispensing end piece (14) on the neck (38) of the reservoir (12), the internal skirt (40) of the dispensing end piece (14) comes into contact with the internal surface (42) of the neck (38) of the reservoir (12) first at the sealing zone (46) and then at the chips retention zone (50).

**4.** Device (10) as claimed in the preceding claim, wherein

- the neck (38) of the reservoir (12) has a length L$_{neck\ min}$ (L5) delimited by the upper end of the length of the neck providing sealing L$_{neck\ sealing}$ (L1) and the upper end of the length of the neck providing the chips retention zone (50) L$_{neck\ retention}$ (L3),
- the dispensing end piece (14) has a length L$_{end\ piece\ max}$ (L6) delimited by the lower end of the length of the end piece providing sealing L$_{end\ piece\ sealing}$ (L2) and the lower end of the length of the end piece providing the chips retention zone (50) L$_{end\ piece\ retention}$ (L4),

and wherein L$_{neck\ min}$ > L$_{end\ piece\ max}$ (L5 > L6).

5. Device (10) as claimed in any one of the preceding claims, wherein the internal surface (42) of the neck (38) of the reservoir (12) at the sealing zone (46) is substantially plane, sealing with the internal skirt (40) of the dispensing end piece (14) being realised by plane-on-plane contact of the internal skirt (40) of the dispensing end piece (14) with the internal surface (42) of the neck (38) of the reservoir (12).

6. Device (10) as claimed in any one of claims 1 to 2, wherein the internal surface (42) of the neck (38) of the reservoir (12) at the sealing zone (46) comprises an annular bead (44).

7. Device (10) as claimed in any one of the preceding claims, wherein the internal surface (42) of the neck (38) of the reservoir (12) comprises, downstream and in the direct vicinity of the sealing zone (46), an annular recess (52).

8. Device (10) as claimed in any one of the preceding claims, wherein the neck (38) of the reservoir (12) is made of low density polyethylene (LDPE) and the internal skirt (42) of the dispensing end piece (14) is made of high density polyethylene (HDPE).

9. Device (10) as claimed in any one of the preceding claims, wherein the end of the internal skirt (40) of the dispensing end piece (14) situated on the side facing the reservoir (12) has a funnel shape.

**Fig. 1**

**Fig.2**

**Fig. 3**

**Fig. 4**

Fig. 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2980378

- US 2011297703 A